# EUROPÄISCHE PATENTSCHRIFT

(11) **EP 3 634 323 B1**
(45) Veröffentlichungstag und Bekanntmachung des Hinweises auf die Patenterteilung: **17.03.2021**
(21) Anmeldenummer: 18742395.9
(22) Anmeldetag: 06.06.2018
(51) Int. Cl.: A61F 5/02

(54) **VORRICHTUNG ZUR VERWENDUNG ALS THERAPIEMITTEL ZUR THERAPEUTISCHEN BEHANDLUNG VON KAMPTOKORMIE**
DEVICE FOR USE AS A THERAPEUTIC MEANS IN THERAPEUTIC TREATMENT OF CAMPTOCORMIA
DISPOSITIF POUR L'UTILISATION COMME MOYEN THERAPEUTIQUE POUR LE TRAITEMENT DE LA CAMPTOCORMIE

(30) Priorität: 08.06.2017 DE 202017002987 U; 21.08.2017 DE 102017119103
(43) Veröffentlichungstag der Anmeldung: 15.04.2020
(73) Patentinhaber: Reha-Team Perick GmbH, 48565 Steinfurt (DE)
(72) Erfinder: PERICK, Jürgen, 48565 Steinfurt (DE)
(74) Vertreter: Schmelcher, Thilo
(86) Internationale Anmeldenummer: PCT/DE2018/100539
(87) Internationale Veröffentlichungsnummer: WO 2018/224096

(56) Entgegenhaltungen:
- US-A- 4 239 211
- US-A- 5 704 530
- US-A- 5 713 840
- US-A1- 2004 143 204
- US-A1- 2009 291 813
- US-A1- 2010 256 717

## Beschreibung

Die Erfindung betrifft eine Vorrichtung zur Verwendung als Therapiemittel zur therapeutischen Behandlung der Kamptokormie.

### Stand der Technik

US5713840 beschreibt eine Vorrichtung in Form eines Gurtzeugs, welche Druck auf den Lendenbereich eines Benutzers ausübt, und welche eine Schwerkraft ausübt, um die Wirbelsäule eines Benutzers in eine aufrechte Position zu drücken. Dadurch ist eine Gewichtsbelastung des Körpers mit einem Gegengewicht auf dem Rücken ein Drehmoment erzeugbar, welches demjenigen welches beispielsweise durch ausgestreckte Arme hervorgerufen wird, entgegenwirkt, um die Wirbelsäule aufrecht zu erhalten.

Als eines der Leitsyndrome der Parkinson-Krankheit gilt die Rigidität, bzw. Muskelsteifheit, welche sich unter anderem in Form einer gekrümmten Körperhaltung auswirkt, welche auch als Kamptokormie bezeichnet wird.

Dabei können als Begleiterscheinung der Kamptokormie Schluckstörungen (Dysphargie) und somit grundlegende Probleme bei der Nahrungs- und Flüssigkeitsaufnahme sowie Atemprobleme auftreten.

Eine Behandlung der Kamptokormie erfolgt zum einen indirekt durch eine pharmazeutische Behandlung der Grunderkrankung, sowie zum anderen direkt, mittels Botulinumtoxin, durch Physiotherapie, durch Ergotherapie, durch Akkupunktur oder durch Massagen. Auch die Behandlung mittels THS, Tiefer Hirnstimulation, ist bekannt.

Insgesamt gilt die Behandlung des Parkinson-Syndroms Kamptokormie als schwierig.

### Offenbarung der Erfindung

Aufgabe der vorliegenden Erfindung ist die Angabe eines neuen Therapiemittels zur therapeutischen Behandlung der Kamptokormie, welche ein Parkinson-Syndrom darstellt.

Erfindungsgemäß vorgesehen ist eine Vorrichtung (1) zur Verwendung als Therapiemittel zur therapeutischen Behandlung einer Kamptokormie, dadurch gekennzeichnet, dass die Vorrichtung (1) zumindest zwei Druckgeber (2) aufweist, welche zur Abgabe von Druckimpulsen auf Kontaktpunkte auf dem linken und dem rechten Brustmuskel eines Patienten anordenbar und einrichtbar sind, wobei die Druckgeber (2) in Form von Trägern (3) eines Gurtzeugs ausgeführt sind, das Gurtzeug Mittel (6) zur Einstellung des Abstands der Träger (3) aufweist und wobei die Träger (3) in Form von Schultergurten zum Umschließen eines Schultergelenks ausgeführt sind, dadurch charakterisiert, dass die Träger (3) eine gebogene Form aufweisen, wie in Anspruch 1 beschrieben.

Vorteilhaft ist durch eine derartige Vorrichtung eine Abgabe von Druckimpulsen auf bestimmte Regionen der Brustmuskel, welche hier als Kontaktpunkte bezeichnet werden, ausübbar, wobei vielfach durch wiederholte Einwirkung von Druckimpulsen auf die Kontaktpunkte am Brustmuskel eine Entspannung der Rumpfbeugemuskulatur erzielbar ist.

Ein Vorteil einer derartigen Vorrichtung ist die Möglichkeit einer beispielsweise ergänzenden, vergleichsweise schonenden Unterstützung einer Kamptokormie-Behandlung mit einem Hilfsmittel mit geringen bis keinen stofflichen Nebenwirkungen, insbesondere im Vergleich zur Verwendung pharmazeutischer Wirkstoffe, sowie einem vergleichsweise geringen Bedarf von medizinischem Personal.

Die wiederholte Stimulation von Kontaktpunkten auf den Brustmuskeln, konkret die wiederholte Ausübung von Druckimpulsen auf bestimmte Regionen der Brustmuskeln, insbesondere durch Druckgeber in Form von Trägern eines Gurtzeugs, hat die überraschende Wirkung gezeigt, einer Kamptokormie entgegen wirken zu können.

Vorteilhaft weist eine derartige Vorrichtung Mittel zur Positionierung der Druckgeber auf Kontaktpunkte im oberen Bereich der Brustmuskel auf, da die Kontaktpunkte patientenindividuell unterschiedlich lokalisiert sein können. Darüberhinaus können die Vorrichtung sowie die Positionierungsmittel am Körper des Patienten tragbar sein. In dieser Ausgestaltung ist es den Patienten möglich, die Vorrichtung über einen längeren Zeitraum, unabhängig von einer stationären oder ambulanten Behandlung, über längere Wirkzeiträume mit sich zu führen.

Dabei zeigen Ergebnisse einer ersten internen Studie an einer kleinen Gruppe von etwa 25 Patienten überraschend positive Ergebnisse. So konnten bei 60% bis 70% der Probanden eine Linderung der Beschwerden festgestellt werden. Diese äußern sich vielfach in einer erkennbar verbesserten Aufrichtung. Auch eine bessere Atmung durch eine Weitung des Brustkorbs sowie eine Linderung von Schluckbeschwerden bei der Nahrungs- und Flüssigkeitszufuhr konnte festgestellt werden. Die guten Ergebnisse an der kleinen, internen Probandengruppe haben zur Planung von klinischen Studien geführt.

Die Positionierungsmittel können beispielsweise in Form eines speziellen Kleidungsstücks oder als Gurtzeug ausgeführt, oder die Druckgeber beispielsweise in Form von Trägern eines Gurtzeugs ausgeführt sein. Eine derartige Ausgestaltung erlaubt den Patienten nicht nur Mobilität, sondern auch Diskretion, da die Vorrichtung bei entsprechender Ausführung auch unter der Kleidung getragen werden kann.

Vorteilhaft sind die Druckgeber in Form von Trägern eines Gurtzeugs ausgeführt. Weiterhin vorteilhaft weisen die Träger eine gebogene Form auf. Dabei können die Träger in Form von Schultergurten zum Umschließen eines Schultergelenks ausgeführt sein.

Durch eine gebogene Form können die zur Entspannung der Muskulatur zu treffenden Kontaktpunkte mit einer großen Wahrscheinlichkeit überdeckt werden. Dies ist mit einer geradlinigen Ausführung der Träger nur sehr viel schwerer oder überhaupt nicht zuverlässig zu erreichen. Durch eine gebogene Form der Träger sind diese zudem einfach und durch ihre Formgebung einen guten Halt bietend um ein Schultergelenk herumführbar. Dabei können die Träger trennbar oder derart gestaltet sein, dass eine unter Beteiligung der Träger ausgebildete Schlaufe zum Umschließen eines Schultergelenks auf einen Umfang von wenigstens 100 cm zu verlängern bzw. aufzuweiten ist.

Eine derartige Ausgestaltung bietet den Vorteil, dass generell unter einer Versteifung der Muskulatur leidende Patienten den Träger ohne größere Mühen überstreifen können. Vorteilhaft ist die Schlaufe auf einen Umfang von 100 cm bis 120 cm zu verlängern, so dass die Schlaufe auch um einen nahezu gestreckten Arm herumführbar ist. Zur Verringerung der Schlaufenweite weist die Vorrichtung vorteilhaft Feststellmittel zum Feststellen von gestrafften Trägern auf. Weiterhin vorteilhaft können die Träger ein Greifende aufweisen, welches im geweiteten Zustand der Schlaufe gut greifbar ist. Da Parkinson-Patienten der Pinzettengriff oftmals schwerfällt, weist ein gut greifbares Greifende als Ende eines Trägers wenigstens eine Länge ca. 10 cm auf. Damit die Schlaufe bei einem Aufweiten nicht unbeabsichtigt geöffnet wird, kann die Vorrichtung eine Sicherheitsvorrichtung aufweisen, durch welche eine Bereitstellung eines Greifendes absicherbar ist.

Die Sicherung kann in Form einer Durchrutschsicherung, insbesondere einer Niete, einer Klemme oder eines Knopfes ausgeführt sein, welche beispielsweise aufgebracht auf einen Träger ein Durchrutschen des Trägerendes durch eine Schnalle verhindert. Alternativ kann die Sicherheitsvorrichtung auch als Schlaufe, als Griff, oder als Karabiner ausgeführt sein.

Weisen die um die Schultergelenke herumgeführten Träger eine gebogene Form und eine Breite von ca. 5-8 cm auf, so werden die zur Entspannung der Muskulatur zu treffenden Kontaktpunkte bereits mit einer großen Wahrscheinlichkeit überdeckt. Um die Kontaktpunkte mit größerer Sicherheit zu treffen, kann die Vorrichtung vorteilhaft Mittel zur exakten Einstellung des Abstands der Träger, zum Beispiel einen Quergurt aufweisen, durch den der Abstand der Träger brustseitig einstellbar ist. Dazu kann der Quergurt mit den Trägern verbindbar sein. In einer vorteilhaften Ausgestaltung sind die Verbindungen des Quergurts mit den Trägern höhenverstellbar.

Vorteilhaft ist die Vorrichtung derart ausgeführt, dass sie der Patient bei ihrer Verwendung auf den Schultern sowie auf dem Rücken kaum, oder im Idealfall, ggf. nach einer Gewöhnungsphase, überhaupt nicht wahrnimmt. Dazu können die Träger im Schulter- und Rückenbereich vorteilhaft flächig ausgeführt sein. In einer vorteilhaften Ausgestaltung der Erfindung weisen die Träger im Schulter- und Rückenbereich eine Breite von ca. 5-8 cm auf.

Die Vorrichtung kann im Bereich der Brustmuskeln sowie im Schulter- und Rückenbereich ähnlich oder gleich breite oder auch unterschiedlich breite Auflageflächen aufweisen.

Für die Wirksamkeit der Vorrichtung ist es erforderlich, dass die auf die Kontaktpunkte an den Brustmuskeln eingestellten Träger nicht verrutschen. Dies kann zumindest teilweise durch eine flächige Ausführung der Träger im Rückenbereich erzielt werden. Vorteilhaft weisen die Träger im Rückenbereich eine gewisse Steifigkeit auf, die derart gewählt ist, dass sie Bewegungen um die Längsachse des Körpers, insbesondere Drehungen, sowie um die Querachse des Körpers, insbesondere Beugungen, kaum oder überhaupt nicht behindert, und eine Stauchung entlang der Querachse des Körpers spürbar behindert.

Zu diesem Zweck können die Träger im Rückenbereich flächig, flächig und miteinander verbunden, und/oder mehrschichtig ausgeführt sein. In einer besonderen Ausführungsform können die Träger beispielsweise eine Einlage aufweisen, welche die Träger miteinander verbindet und den Abstand und/oder Winkel zwischen den Trägern aussteift.

Die Einlage ist durch Form- und/oder Materialwahl vorteilhaft derart beschaffen, dass sie mit mäßigem, beim Tragen etwas spürbaren Kraftaufwand auch eine Verformung in den Aussteifungsrichtungen zulässt. Mögliche Ausführungsformen der Einlage können beispielsweise ein steifer textiler Stoff oder eine dünne Kunststoffeinlage sein. Alternativ zu einer Einlage kann auch das Trägermaterial selbst diese Eigenschaften aufweisen.

In einer vorteilhaften Ausgestaltung der Vorrichtung weist die Vorrichtung eine rückseitig angeordnete Rückentasche auf. Dabei ist unter einer rückseitigen Anordnung der Rückentasche eine Lokalisierung zu verstehen, welche bedeutet, dass die Rückentasche bei einer Mitführung der Vorrichtung durch den Patienten auf dessen Rücken angeordnet ist. Vorteilhaft erfolgt die Anordnung derart, dass durch die Positionierung und/oder die Formgebung der Rückentasche eine Entstehung von Kontaktpunkten oder Druckpunkten am Rücken des Patienten verhindert bzw. minimiert wird.

Eine derartige Ausgestaltung erhöht nicht nur den Tragekomfort, sondern verhindert die Einwirkung zusätzlicher Reize auf den Patienten, welche von den durch die Druckgeber ausgehenden Reize ablenken und so den Therapieerfolg mindern und/oder gefährden.

Vorteilhaft ist die Rückentasche zur mittigen Positionierung auf dem Rücken mit den Trägern verbunden. Dazu kann sie beispielsweise mit diesen vernäht sein.

Weiterhin vorteilhaft sind die Träger derart in ihrer Länge einstellbar, dass die Rückentasche an einem Patienten mittig unterhalb der Schulterblätter positionierbar ist. Dazu können die Träger in Form eines V, eines U oder eines Bogens zusammengeführt sein und damit die vertikale Lokalisierung auf dem Rücken vorgeben. Vorteilhaft weisen die Träger dazu in diesem Bereich eine Breite von ca. 5-8 cm auf, wodurch ein flächiges Anliegen am Körper unterstützt und die Gefahr eines Verdrehens oder Verrutschens gemindert wird.

In einer vorteilhaften Ausgestaltung der Erfindung sind die Träger V-förmig zueinander angeordnet und weisen einen Öffnungswinkel zwischen 45 Grad und 55 Grad, insbesondere von 50 Grad auf. Für durchschnittlich proportionierte Patienten erlaubt dieser Winkel größenunabhängig sowohl eine gute Positionierung der Träger auf der Schulter, um die Kontaktpunkte auf den Brustmuskeln zu treffen, als auch die gewünschte Positionierung einer Rückentasche unterhalb der Schulterblätter.

In einem über der Schulter zu liegendem Bereich weisen die Träger vorteilhaft eine leichte Biegung nach innen in Richtung des Brustbeins auf. Vorteilhaft weisen die Träger im Schulterbereich einen mittleren Krümmungsradius R1 zwischen 40 cm und 45 cm, insbesondere von 43 cm auf.

In einem über der Brust zu liegendem Bereich weisen die Träger vorteilhaft eine leicht nach außen gerichtete, das Schultergelenk teilweise umschließend und den Brustmuskel überdeckende Biegung auf. Vorteilhaft weisen die Träger im Brustmuskelbereich einen mittleren Krümmungsradius R2 zwischen 25 cm und 30 cm, insbesondere von 28 cm auf.

Vorteilhaft weisen die Träger eine gebogene S-Form auf. Gemäß vorangegangenen Ausführungen weisen die Träger vorteilhaft sowohl eine Krümmung nach innen als auch eine Krümmung nach außen auf. Bei einer Sicht auf ein geöffnetes, flachgestrecktes Gurtzeug aus rückwärtiger Perspektive beschreibt der Verlauf des linken Trägers vom Rücken zur Brust die Form eines "S", und der Verlauf des rechten Trägers von Brust zum Rücken ein "S".

Weiterhin vorteilhaft kann der auf dem Rücken verlaufenden Trägeranteil des Trägers in seiner Länge verstellbar sein. Dies kann erforderlich sein, um die richtige Positionierung eines gebogenen Teils des Trägers auf den Brustmuskeln auch bei sehr kleinen oder sehr korpulenten Patienten zu gewährleisten. Alternativ könnte die Vorrichtung mit Trägern in unterschiedlichen Größen verbindbar sein. Um den unterschiedlichen Größenanforderungen der Patienten gerecht zu werden kann es weiterhin erforderlich sein, den Radius R1 aus einem Bereich von 20 cm bis 35 cm, und den Radius R2 aus einem Bereich von 35 cm bis 50 cm zu wählen.

Weiterhin vorteilhaft ist die Rückentasche flach und/oder kissenförmig ausgebildet, wobei sie vorteilhaft eine Höhe von 3 cm nicht übersteigt. In einer vorteilhaften Ausgestaltung weist die Rückentasche eine Höhe von weniger als 2 cm auf. Eine derartige Ausgestaltung ermöglicht ein relatives bequemes und unauffälliges Tragen der Vorrichtung mit der Rückentasche unter der Kleidung.

Die Rückentasche ist zur Aufnahme eines flächigen Gewichts ausgebildet. Dieses kann fest mit der Rückentasche verbunden, beispielsweise eingenäht sein.

Vorteilhaft aber ist die Rückentasche zur alternativen, und jeweils passgenauen Aufnahme von ein bis zu drei flächigen Gewichten zu 0,25 kg bis 0,75 kg, 0,5 kg bis 1,5 kg und 0,5 kg bis 2 kg ausgeführt.

Die Gewichtskraft der flächigen Gewichte ist geeignet, eine Zugkraft auf die Träger der Vorrichtung auszuüben, welche diese in Form eines Drucks auf den Körper des Patienten ableiten. Dabei führt eine Bewegung des Patienten auf Grund der Massenträgheit zu Druckschwankungen, die sich beispielsweise bei einem Gehen in Form von Druckimpulsen auswirken. Somit sind die Druckgeber der Vorrichtung in dieser vorteilhaften Ausgestaltung durch die Bewegung des Patienten antreibbar. Alternativ oder ergänzend kann die Vorrichtung einen elektrischen Druckgeber aufweisen. Ferner kann die Vorrichtung eine Energiequelle aufweisen, beispielsweise in Form eines flachen Bleiakkumulators welcher alternativ oder ergänzend zu einem Gewicht in der Rückentasche anordenbar ist.

Eine alternative Befüllung der Rückentasche mit einem, zwei oder drei Gewichten erlaubt die Stärke der Impulse patientenindividuell einstellen zu können, wobei die Ausführung der Gewichte von 0,25 kg bis 0,75 kg, 0,5 kg bis 1,5 kg und 0,5 kg bis 2 kg zusammen mit den Kombinationsmöglichkeiten einen sinnvollen Gewichtsbereich abdeckt. Dieser vergleichsweise schmale Gewichtsbereich ist in der Regel für die Wirksamkeit der Vorrichtung ausreichend, da eine erwünschte Aufrichtung des Körpers nicht durch eine mechanische Hebelwirkung der nach hinten ziehenden Gewichte, sondern durch die auf die Brustmuskeln abgegebenen Druckimpulse bewirkt wird. In einer vorteilhaften Ausführung sind die Gewichte in einem Satz zu 0,5 kg, 1kg und 1 kg ausgeführt. Weiterhin vorteilhaft weisen sie passgenau das Maß der Rückentasche und abgerundete Ecken auf und sind in Folie verschweißt. Eine passgenaue Anpassung an das Maß der Rückentasche verhindert eine Bewegung der Gewichte in der Rückentasche und damit die unbeabsichtigte Entstehung von Störimpulsen.

Weiterhin vorteilhaft kann die Rückentasche der Vorrichtung eine annähernd rechteckige Grundfläche aufweisen. Dies kann vorteilhaft eine Grundfläche von 15 cm - 19 cm Breite x 17 cm - 22 cm Länge sein, wobei die Längsseite bei einer Benutzung parallel zur Wirbelsäule des Patienten orientiert ist. Vorteilhaft weist die Rückentasche ein Außenmaß von 17 cm x 19,5 cm auf. Eine derart dimensionierte Rückentasche bietet den Vorteil, dass sie klein genug ist, um bei Patienten unterschiedlicher Größe verwendbar zu sein, und groß genug ist, um flächige Gewichte mit einer ausreichend großen Masse aufnehmen zu können.

Alternativ zu einer rechteckigen, plan ausgeführten Rückentasche kann diese auch an die Anatomie des Körpers angepasst ausgeführt sein, beispielsweise eine Wölbung zur Anpassung an die Anatomie des Körpers aufweisen. Weiterhin kann sie eine Mulde oder Rinne für die Wirbelsäule und/oder von einem Rechteck abweichende Grundformen aufweisen.

In einer vorteilhaften Ausgestaltung der Vorrichtung weist die Rückentasche auf ihrer zum Rücken eines Patienten weisenden Seite einen ersten viskoelastischen Schaumstoff auf. Vorteilhaft kann dieser derart dimensioniert und angeordnet sein, dass die Gewichte in einer teilweise befüllten Rückentasche durch den Schaumstoff fixierbar sind. Dabei ist der viskoelastischen Schaumstoff vorteilhaft derart dimensioniert und angeordnet, dass er im entspannten Zustand den Innenraum der Rückentasche im Wesentlichen ausfüllt.

In einer weiteren vorteilhaften Ausgestaltung der Vorrichtung weist die Rückentasche einen zweiten viskoelastischen Schaumstoff auf, welcher derart angeordnet ist, dass zur Verhinderung von Kontaktpunkten bzw. Druckpunkten eine durch die Gewichte in der Rückentasche ausübbare Andruckkraft auf den Rücken des Patienten flächig verteilt wird, um damit ebenfalls die unbeabsichtigte Entstehung von Störimpulsen zu unterdrücken.

In einer vorteilhaften Ausgestaltung der Vorrichtung sind der erste und der zweite viskoelastische Schaumstoff einstückig ausgeführt.

Weiterhin vorteilhaft ist die Rückentasche verschließbar. Ein derartiger Verschluss verhindert, dass die Gewichte insbesondere bei einer Bückbewegung aus der Rückentasche herausrutschen. Dabei kann der Verschluss vorteilhaft als Klettverschluss ausgeführt sein. Bei einer vorteilhaften Anordnung des Klettverschlusses ist dieser auf der Innenseite der Rückentasche derart angeordnet, dass die Seitenkante des Klettverschlusses direkt an einem in die Tasche aufnehmbaren Gewicht anliegt.

Desweiteren kann die Vorrichtung vorteilhaft einen mechanischen Impulswandler aufweisen, welcher geeignet ist, einen von einem Druckgeber abgebbaren flächigen Druckimpuls auf einen oder mehrere punktuelle Druckimpulse umzusetzen. Auf diese Weise können die Druckimpulse verstärkt, bzw. die Gewichte reduziert werden. Dabei ist unter einem punktuell wirkenden Druckimpuls ein Druckimpuls zu verstehen, welcher auf eine Fläche von wenigen Quadratmillimetern bis hin zu einigen Quadratzentimetern einwirkt. Weiterhin kann der Impulswandler in Form von einem oder mehreren, unter einem Druckgeber anordenbarer Noppen ausgeführt sein.

Offenbart ist ferner die Verwendung einer Vorrichtung mit zwei Druckgebern zur Abgabe von Druckimpulsen auf den linken und rechten Brustmuskel eines Patienten als therapeutische Vorrichtung, insbesondere für einen Parkinson-Patienten, wobei, wie aus den vorangehenden Ausführungen ersichtlich, die Wirkung der Vorrichtung in der Vorrichtung selbst begründet ist.

Offenbart ist weiterhin die Verwendung einer Vorrichtung zur Verwendung als Therapiemittel zur therapeutischen Behandlung eines Parkinson-Syndroms, welche zumindest zwei Druckgeber aufweist, welche zur Abgabe von Druckimpulsen auf Kontaktpunkte auf dem linken und dem rechten Brustmuskel eines Patienten anordenbar und zur Abgabe von Druckimpulsen einrichtbar sind.

In einer vorteilhaften Ausführungsform ist eine Vorrichtung zur Verwendung als Therapiemittel zur therapeutischen Behandlung eines Parkinson-Syndroms eine therapeutische Vorrichtung zur Behandlung der Kamptokormie, beispielsweise in Form eines Kamptokormie-Rucksacks.

In einer vorteilhaften Ausführungsform betrifft die Erfindung einen Kamptokormie-Rucksack mit gebogenen Schultergurten zur therapeutischen Unterstützung der Parkinsontherapie, wobei die auch als Träger bezeichneten Schultergurte eine gebogene Form aufweisen, in ihrer Länge verstellbar sind, und der Kamptokormie-Rucksack eine flache Rückentasche zur Aufnahme von flachen Gewichten und einen brustseitigen Quergurt zur exakten Einstellung der Träger aufweist, und wobei zwecks Erzielung einer therapeutischen Wirkung der Kamptokormie-Rucksack wie folgt angelegt wird:
- die flache Rückentasche wird über die Einstellung der Träger mittig und zu den Schulterblättern berührungslos auf dem Rücken eines Patienten positioniert und brustseitig mittels des Quergurts der Abstand der gebogenen Träger eingestellt,
- wobei die gebogenen Träger Kontaktpunkte auf den Brustmuskeln überdecken
- und Druckimpulse auf die Kontaktpunkte abgegeben werden infolge einer Bewegung des Patienten.

In weiteren Ausführungsformen kann der Kamptokormie-Rucksack weitere der vorbeschriebenen Ausgestaltungsmerkmale der Vorrichtung zur Verwendung als Therapiemittel zur therapeutischen Behandlung eines Parkinson-Syndroms aufweisen.

### Zeichnungen

Nachfolgend wird die Erfindung unter Bezugnahme auf die anliegenden, schematischen Zeichnungen anhand bevorzugten Ausführungsformen näher erläutert.

Es zeigen
- Fig. 1: eine Skizze eines Kamptokormie-Rucksacks als bevorzugte Ausführung einer Vorrichtung zur Verwendung als Therapiemittel zur therapeutischen Behandlung eines Parkinson-Syndroms;
- Fig. 2: eine Skizze eines Kamptokormie-Rucksacks mit ausgebreiteten Trägern mit gebogener Form.

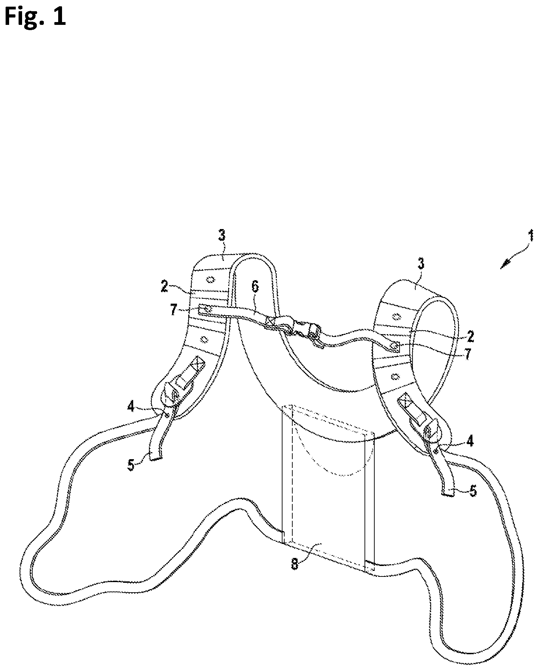

Fig. 1 zeigt eine Skizze eines Kamptokormie-Rucksacks 1 in perspektivischer Darstellung.

Aus Fig. 1 ist ein Kamptokormie-Rucksack 1 als bevorzugte Ausführung einer Vorrichtung 1 zur Verwendung als Therapiemittel zur therapeutischen

Behandlung eines Parkinson-Syndroms ersichtlich, wobei die Vorrichtung 1 zwei Druckgeber 2 aufweist, welche zur Abgabe von Druckimpulsen auf Kontaktpunkte auf dem linken und dem rechten Brustmuskel eines Patienten anordenbar und einrichtbar sind.

Dabei sind die Druckgeber 2 in Form von Trägern 3 eines Gurtzeugs ausgeführt. Das Gurtzeug weist Mittel 6 zur Einstellung des Abstands der Träger 3 auf, wobei die Mittel 6 zur Einstellung des Abstands der Träger 3 mit Hilfe von Mitteln 7 in der Höhe verstellbar sind. Die Träger 3 weisen eine gebogene Form auf und sind in Form von Schultergurten zum Umschließen eines Schultergelenks ausgeführt.

Eine unter Beteiligung der Träger 3 ausgebildete Schlaufe zum Umschließen eines Schultergelenks ist auf einen Umfang von wenigstens 100 cm aufzuweiten. Die Träger 3 weisen ein Greifende 5 auf, welches im aufgeweiteten Zustand der Schlaufe gut greifbar ist, d.h. eine Länge von ca. 10 cm aufweist. Der Kamptokormie-Rucksack 1 weist eine Sicherheitsvorrichtung 4 auf, durch welche eine Bereitstellung eines Greifendes 5 absicherbar ist.

Weiterhin aus Fig. 1 ersichtlich weist der Kamptokormie-Rucksack 1 eine rückseitig angeordnete flache Rückentasche 8 auf, welche eine Höhe von weniger als 3 cm aufweist. Die Rückentasche 8 weist eine derartige Formgebung auf, dass eine Entstehung einzelner Kontaktpunkte am Rücken des Patienten, insbesondere an den Schulterblättern, vermieden wird. Die Träger 3 sind derart in ihrer Länge einstellbar, dass die Rückentasche 8 an einem Patienten mittig unterhalb der Schulterblätter positionierbar ist.

Die Rückentasche 8 ist zur alternativen passgenauen Aufnahme von ein bis zu drei flächigen Gewichten 0,5 kg, 1 kg und 1 kg ausgeführt und weist eine annähernd rechteckige Grundfläche von 17 cm Breite x 19,5 cm Länge auf.

Nicht dargestellt weist die Rückentasche 8 auf ihrer zum Rücken eines Patienten weisenden Seite einen viskoelastischen Schaumstoff auf. Der viskoelastische Schaumstoff auf der zum Rücken eines Patienten weisenden Seite ist derart dimensioniert und angeordnet, dass die Gewichte in einer teilweise befüllten Rückentasche durch den Schaumstoff fixierbar sind.

Befinden sich keine Gewichte in der Rückentasche 8, füllt der viskoelastische Schaumstoff im entspannten Zustand den Innenraum der Rückentasche 8 im Wesentlichen, d.h. nahezu vollständig aus. Ferner ist der Schaumstoff derart angeordnet, dass zur Verhinderung von Kontaktpunkten eine durch die Gewichte ausübbare Andruckkraft der Rückentasche 8 auf den Rücken des Patienten flächig verteilt wird.

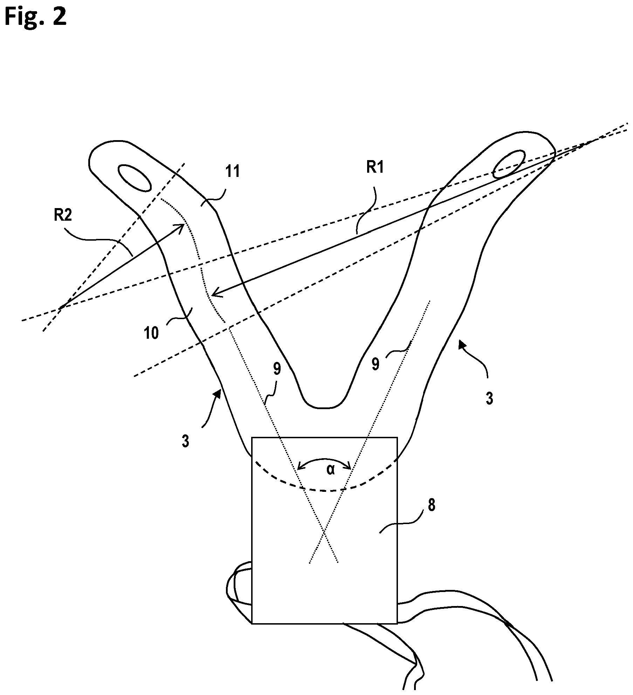

Fig. 2 zeigt eine Skizze eines Kamptokormie-Rucksacks mit ausgebreiteten Trägern, wobei die Träger eine gebogene Form aufweisen.

Die Träger 3 sind V-förmig zueinander angeordnet und weisen einen Öffnungswinkel α zwischen 45 Grad und 55 Grad, konkret von 50 Grad auf. Dabei liegt ein fiktiver Schnittpunkt der Trägermittellinien 9 etwa mittig in der Rückentasche 8. In einem über der Schulter zu liegendem Bereich 10 weisen die Träger 3 vorteilhaft eine leichte Biegung nach innen in Richtung des Brustbeins auf. Der mittlere Krümmungsradius R1 der Träger 3 liegt in diesem Bereich 10 zwischen 40 cm und 45 cm, konkret beträgt er 43 cm. In einem über der Brust zu liegendem Bereich 11 weisen die Träger 3 eine leicht nach außen gerichtete, das Schultergelenk teilweise umschließende und den Brustmuskel überdeckende, gebogene Form auf. Die Träger 3 weisen im Bereich 11 einen mittleren Krümmungsradius R2 zwischen 25 cm und 30 cm, konkret von 28 cm auf. Weiterhin aus Fig. 2 ersichtlich weisen die Träger eine gebogene S-Form auf. Bei der dargestellten Sicht auf ein geöffnetes, flachgestrecktes Gurtzeug aus rückwärtiger Perspektive beschreibt der Verlauf des linken Trägers 3 vom Rücken zur Brust, d.h. in Fig. 2 von unten nach oben, die Form eines "S", und der Verlauf des rechten Trägers 3 von Brust zum Rücken, d.h. in Fig. 2 von oben nach unten, ebenfalls ein "S".

## Patentansprüche

1. Vorrichtung (1) zur Verwendung als Therapiemittel zur therapeutischen Behandlung einer Kamptokormie, **dadurch gekennzeichnet, dass** die Vorrichtung (1) zumindest zwei Druckgeber (2) aufweist, welche zur Abgabe von Druckimpulsen auf Kontaktpunkte auf dem linken und dem rechten Brustmuskel eines Patienten anordenbar und einrichtbar sind, wobei die Druckgeber (2) in Form von Trägern (3) eines Gurtzeugs ausgeführt sind, das Gurtzeug Mittel (6) zur Einstellung des Abstands der Träger (3) aufweist und wobei die Träger (3) Form von Schultergurten zum Umschließen eines Schultergelenks ausgeführt sind, dadurch charakterisiert, dass die Träger (3) eine gebogene Form aufweisen.

2. Vorrichtung (1) nach Anspruch 1, **dadurch gekennzeichnet, dass** die Träger (3) trennbar sind oder eine unter Beteiligung der Träger (3) ausgebildete Schlaufe zum Umschließen eines Schultergelenks auf einen Umfang von wenigstens 100 cm aufzuweiten ist.

3. Vorrichtung (1) nach einem der Ansprüche 1 oder 2, **dadurch gekennzeichnet, dass** die Träger (3) ein Greifende (5) aufweisen, welches im aufgeweiteten Zustand einer unter Beteiligung der Träger (3) ausgebildete Schlaufe zum Umschließen eines Schultergelenks gut greifbar ist und die Vorrichtung eine Sicherheitsvorrichtung (4) aufweist, durch welche eine Bereitstellung eines Greifendes (5) absicherbar ist.

4. Vorrichtung (1) nach einem der Ansprüche 1 bis 3, **dadurch gekennzeichnet, dass** die Vorrichtung (1) eine rückseitig angeordnete flache Rückentasche (8) aufweist, welche eine Höhe von weniger als 3 cm aufweist.

5. Vorrichtung (1) nach Anspruch 4, **dadurch gekennzeichnet, dass** die Rückentasche (8) eine derartige Formgebung aufweist, dass eine Entstehung einzelner Kontaktpunkte am Rücken des Patienten vermieden wird.

6. Vorrichtung (1) nach einem der Ansprüche 4 bis 5, **dadurch gekennzeichnet, dass** die Träger (3) derart in ihrer Länge einstellbar sind, dass die Rückentasche (8) an einem Patienten mittig unterhalb der Schulterblätter positionierbar ist.

7. Vorrichtung (1) nach einem der Ansprüche 4 bis 6, **dadurch gekennzeichnet, dass** die Rückentasche (8) in Form eines Kissens ausgeführt ist.

8. Vorrichtung (1) nach einem der Ansprüche 4 bis 7, **dadurch kennzeichnet, dass** die Rückentasche (8) eine annähernd rechteckige Grundfläche von 15 cm - 19 cm Breite x 17 cm - 22 cm Länge aufweist.

9. Vorrichtung (1) nach einem der Ansprüche 4 bis 8, **dadurch kennzeichnet, dass** die Rückentasche (8) auf ihrer zum Rücken eines Patienten weisenden Seite einen ersten viskoelastischen Schaumstoff aufweist.

10. Vorrichtung (1) nach Anspruch 9, **dadurch gekennzeichnet, dass** der erste viskoelastischen Schaumstoff auf der zum Rücken eines Patienten weisenden Seite derart dimensioniert und angeordnet ist, dass die Gewichte in einer teilweise befüllten Rückentasche durch den Schaumstoff fixierbar sind.

11. Vorrichtung (1) nach einem der Ansprüche 9 oder 10, **dadurch kennzeichnet, dass** erste viskoelastische Schaumstoff im entspannten Zustand den Innenraum der Rückentasche (8) im Wesentlichen ausfüllt.

12. Vorrichtung (1) nach einem der Ansprüche 9 bis 11, **dadurch kennzeichnet, dass** die Vorrichtung (1) einen zweiten viskoelastischen Schaumstoff aufweist, welcher derart angeordnet ist, dass zur Verhinderung von Kontaktpunkten eine durch die Rückentasche (8) ausübbare Andruckkraft auf den Rücken des Patienten flächig verteilt wird.

13. Vorrichtung (1) nach Anspruch 12, **dadurch kennzeichnet, dass** der erste und der zweite Schaumstoff einstückig ausgeführt sind.

14. Vorrichtung (1) nach einem der vorherigen Ansprüche, **dadurch gekennzeichnet, dass** die Vorrichtung (1) einen Impulswandler aufweist, welcher geeignet ist, einen von einem Druckgeber (2) abgebbaren flächigen Druckimpuls, auf einen oder mehrere punktuelle Druckimpulse umzusetzen.

15. Vorrichtung (1) nach Anspruch 14, **dadurch kennzeichnet, dass** der Impulswandler in Form von einem oder mehreren, unter einem Druckgeber (2) anordenbaren Noppen ausgeführt ist.

## Claims

1. Device (1) for use as a therapeutic aid for the therapeutic treatment of a Camptocormia, **characterized in that** the device (1) has at least two pressure transducers (2), which can be arranged and set up to deliver pressure impulses to contact points on a patient's left and right pectoral muscles, whereby the pressure transducers (2) are fitted in the form of harness straps (3), the harness has aids (6) to adjust the spacing of the straps (3) and whereby the straps (3) are fitted in the form of shoulder harnesses to enclose the shoulder joint, **characterized in that** the straps (3) have a curved shape.

2. Device (1) according to claim 1, **characterized in that** the straps (3) can be separated or a loop created with the straps (3) to enclose the shoulder joint and widened to a length of at least 100 cm.

3. Device (1) according to one of the claims 1 or 2, **characterized in that** the straps (3) have one grasping end (5), which in the widened state creates an easily grasped loop out of the straps (3) to enclose the shoulder joint and the device has a safety device (4) through which the provision of a grasping end (5) can be protected.

4. Device (1) according to one of the claims 1 to 3, **characterized in that** the device (1) has a flat backside pocket (8) situated on the back side with a height of less than 3 cm.

5. Device (1) according to claim 4, **characterized in that** the backside pocket (8) has been designed in a way to prevent the formation of individual contact points on the patient's back.

6. Device (1) according to one of the claims 4 to 5, **characterized in that** the length of the straps (3) can be adjusted in such a way that the backside pocket (8) can be positioned centrally below the patient's shoulder blades.

7. Device (1) according to one of the claims 4 to 6, **characterized in that** the backside pocket (8) is designed in the form of a pillow.

8. Device (1) according to one of the claims 4 to 7, **characterized in that** the backside pocket (8) has a rough rectangular base surface with a width of 15 - 19 cm x a length of 17-22 cm.

9. Device (1) according to one of the claims 4 to 8, **characterized in that** the backside pocket (8) has a first viscoelastic foam material on the side facing the patient's back.

10. Device (1) according to claim 9, **characterized in that** the first viscoelastic foam material is dimensioned and arranged in such a way on the side facing the patient's back that the weights can be fixed in place in a partially filled backside pocket by the foam material.

11. Device (1) according to one of the claims 9 or 10, **characterized in that** the first viscoelastic foam material largely fills the interior of the backside pocket (8) in a relaxed state.

12. Device (1) according to one of the claims 9 to 11, **characterized in that** the device (1) has a second viscoelastic foam material arranged in such a way that a clamping force exerted on the back of the patient by the backside pocket (8) is flatly distributed to prevent contact points.

13. Device (1) according to claim 12, **characterized in that** the first and second foam material are fitted as one single piece.

14. Device (1) according to one of the former claims, **characterized in that** the device (1) has an impulse converter suitable for converting a flat pressure impulse delivered by a pressure transducer (2) to one or several selective pressure impulses.

15. Device (1) according to claim 14, **characterized in that** the impulse converter is fitted in the form of one or several knobs that can be arranged under a pressure transducer (2).

## Revendications

1. Dispositif (1) destiné à être utilisé comme agent thérapeutique pour le traitement thérapeutique de la camptocormie, **caractérisé en ce que** le dispositif (1) comporte au moins deux transducteurs de pression (2) qui peuvent être agencés et configurés pour délivrer des impulsions de pression à des points de contact sur les muscles thoraciques gauche et droit d'un patient, où les transducteurs de pression (2) se présentent sous la forme de sangles (3) d'un harnais, le harnais comporte des moyens (6) pour régler l'espacement des sangles (3) et les sangles (3) sous forme de bretelles pour envelopper une articulation de l'épaule, **caractérisé en ce que** les sangles (3) ont une forme incurvée.

2. Dispositif (1) selon la revendication 1, **caractérisé en ce que** les sangles (3) sont séparables ou une boucle avec la participation des sangles (3) pour envelopper une articulation de l'épaule doit être étendue jusqu'à une circonférence d'au moins 100 cm.

3. Dispositif (1) selon l'une des revendications 1 ou 2, **caractérisé en ce que** les sangles (3) présentent une extrémité de préhension (5) qui, à l'état déployé, peut être facilement saisie par une boucle formée avec la participation des sangles (3) pour envelopper une articulation de l'épaule et le dispositif comporte un dispositif de sécurité (4) par lequel la fourniture d'une extrémité de préhension (5) peut être assurée.

4. Dispositif (1) selon l'une des revendications 1 à 3, **caractérisé en ce que** le dispositif (1) présente une poche dorsale (8) plate disposée à l'arrière et d'une hauteur inférieure à 3 cm.

5. Dispositif (1) selon la revendication 4, **caractérisé en ce que** la poche dorsale (8) a une forme telle que des points de contact individuels sont évités sur le dos du patient.

6. Dispositif (1) selon l'une des revendications 4 à 5, **caractérisé en ce que** la longueur des sangles (3) peut être réglée de telle sorte que la poche dorsale (8) puisse être positionnée sur un patient au centre sous les omoplates.

7. Dispositif (1) selon l'une des revendications 4 à 6, **caractérisé en ce que** la poche dorsale (8) est réalisée sous la forme d'un coussin.

8. Dispositif (1) selon l'une des revendications 4 à 7, **caractérisé en ce que** la poche dorsale (8) a une surface de base approximativement rectangulaire de 15 cm à 19 cm de large x 17 cm à 22 cm de long.

9. Dispositif (1) selon l'une des revendications 4 à 8, **caractérisé en ce que** la poche dorsale (8) présente une première mousse viscoélastique sur sa face tournée vers le dos d'un patient.

10. Dispositif (1) selon la revendication 9, **caractérisé en ce que** la première mousse viscoélastique est dimensionnée et disposée du côté tourné vers le dos du patient de manière à ce que les poids puissent être fixés dans une poche dorsale partiellement remplie par la mousse.

11. Dispositif (1) selon l'une des revendications 9 ou 10, **caractérisé en ce que** la première mousse viscoélastique à l'état détendu remplit essentiellement l'intérieur de la poche dorsale (8).

12. Dispositif (1) selon l'une des revendications 9 à 11, **caractérisé en ce que** le dispositif (1) comporte une deuxième mousse viscoélastique, qui est disposée de telle sorte que, pour éviter les points de contact, une force de pression exercée par la poche dorsale (8) au dos du patient est répartie sur une grande surface.

13. Dispositif (1) selon la revendication 12, **caractérisé en ce que** la première et la deuxième mousse sont réalisées d'une seule pièce.

14. Dispositif (1) selon l'une des revendications précédentes, **caractérisé en ce que** le dispositif (1) comporte un convertisseur d'impulsions adapté pour convertir une impulsion de pression plate émise par un transducteur de pression (2) en une ou plusieurs impulsions de pression ponctuelles.

15. Dispositif (1) selon la revendication 14, **caractérisé en ce que** le convertisseur d'impulsions est réalisé sous la forme d'un ou plusieurs picots pouvant être disposés sous un transducteur de pression (2).
